# EUROPEAN PATENT APPLICATION

(11) **EP 3 603 549 A1**
(43) Date of publication of application: **05.02.2020**
(21) Application number: 18771917.4
(22) Date of filing: 23.03.2018
(51) Int. Cl.: A61B 17/34, A61M 25/06

(54) **DILATOR**

(30) Priority: 24.03.2017 WO PCT/JP2017/012024
(71) Applicant: Asahi Intecc Co., Ltd., Seto-shi, Aichi 489-0071 (JP)
(72) Inventor: FUSEYA, Yukihiro, Seto-shi, Aichi 489-0071 (JP); MAKI, Hideaki, Seto-shi, Aichi 489-0071 (JP); TAKAHASHI, Daiki, Seto-shi, Aichi 489-0071 (JP); SAWAI, Akira, Seto-shi, Aichi 489-0071 (JP); KITAI, Marina, Seto-shi, Aichi 489-0071 (JP)
(74) Representative: Winter, Brandl, Fürniss, Hübner, Röss, Kaiser, Polte - Partnerschaft mbB
(86) International application number: PCT/JP2018/011671
(87) International publication number: WO 2018/174240

(57) **Abstract**

An object of the present invention is to provide a dilator allowing the completion of hole expansion on a target object to be accurately detected.

A dilator 1 includes a hollow shaft 11 having a tapered portion 111 and a body portion 112 having a front end located at a base end 111b of the tapered portion 111, the tapered portion 111 having an outer diameter of a front end smaller than that of the base end, the body portion 112 extending toward a base end side in an axis direction of the tapered portion 111, in which a spirally-arranged protruding portion 21 is provided on an outer peripheral surface 111A of the tapered portion 111, and the spirally-arranged protruding portion 21 has a gap 21a along an axis direction of the shaft 11, and a base end 21b of the spirally-arranged protruding portion 21 is located at the base end 111b of the tapered portion 111.

## Description

### TECHNICAL FIELD

The present invention relates to a dilator.

### BACKGROUND ART

A dilator is known, for example, as a device for expanding a hole opened on the wall of an organ such as stomach and liver, a narrowed segment in a body cavity such as bile duct and pancreatic duct, and the like.

Such a dilator usually includes a tapered portion for expanding a pre-opened hole, the tapered portion having a diameter gradually increasing from the front end toward the base end and being disposed at a portion of a shaft. Here, during the procedure, the front end of an endoscope is first positioned in the vicinity of a site where a hole is to be opened, and a needle is allowed to advance through the front end of the endoscope to open a hole on the wall of an organ and the like. Then a guide wire is inserted into the hole through an through-hole of the needle, and then the needle is withdrawn. Subsequently, the dilator is delivered to the hole along the guide wire, and pushed into the hole to expand the hole by the aforementioned tapered portion.

In view of the above circumstances, for example, a dilator including a contrast maker disposed in the vicinity of a hole-expanding tapered portion is disclosed as a technology for determining the position of a dilator inside the body (for example, see Patent Literature 1).

According to the above technology, the position of a contrast maker can be perceived under X-ray imaging, enabling the position of the tapered portion inside the body to be approximately detected during the procedure.

### CITATION LIST

### Patent Literature

Patent Literature 1: Japanese Patent Application Laid-Open No 2017-51328

### SUMMARY OF INVENTION

### TECHNICAL PROBLEM

Nonetheless, the completion of hole expansion with the tapered portion is difficult to be accurately detected in the conventional dilator as described above although the position of the tapered portion during the procedure can be approximately detected. This may result in insufficient insertion of the dilator and thus insufficient expansion of a hole, or may result in excessive pushing-in of the dilator.

The present invention is made in view of the above circumstances. An object of the present invention is to provide a dilator allowing the completion of hole expansion on a target object to be accurately detected.

### SOLUTION TO PROBLEM

Several aspects of the present disclosure include the followings.
(1) A dilator including a hollow shaft having a tapered portion and a body portion having a front end located at a base end of the tapered portion, the tapered portion having an outer diameter of a front end smaller than that of the base end, the body portion extending toward a base end side in an axis direction of the tapered portion,
   wherein,
   a spirally-arranged protruding portion is provided on an outer peripheral surface of the tapered portion, and
   the spirally-arranged protruding portion has a gap along an axis direction of the shaft, and
   a base end of the spirally-arranged protruding portion is located at the base end of the tapered portion.
(2) The dilator according to (1), wherein the outer shape of the spirally-arranged protruding portion in a front view in the axis direction of the tapered portion falls within the outer shape of the body portion in a front view in the axis direction of the tapered portion in a state where the tapered portion and the body portion extend in a substantially linear fashion.
(3) The dilator according to (1) or (2), wherein a material of the spirally-arranged protruding portion includes a radiopaque material.
(4) The dilator according to any one of (1) to (3), wherein the shaft includes a front end portion having a base end located at the front end of the tapered portion and extending toward a front end side in the axis direction of the tapered portion.
(5) The dilator according to (4), wherein a front end of the spirally-arranged protruding portion is located at the front end of the tapered portion or an outer peripheral surface of the front end portion.
(6) The dilator according to any one of (1) to (5), wherein the shaft is formed such that a wire is wound around an axis of the shaft.
(7) The dilator according to any one of (1) to (6), wherein the spirally-arranged protruding portion is formed such that a wire is wound around on an outer peripheral surface of the shaft.
(8) The dilator according to any one of (1) to (5), wherein the shaft is formed such that a wire is wound around the axis of the shaft, and the spirally-arranged protruding portion is formed such that a wire is wound around on the outer peripheral surface of the shaft, and
   a winding direction of the wire of the shaft and a winding direction of the wire of the spirally-arranged protruding portion are opposite to each other.

It is noted that the term "front end side" as used herein refers to a direction along the axis direction of the shaft in which the tapered portion is located with respect to the body portion. Further, the term "base end side" refers to a direction along the axis direction of the dilator which is opposite to the front end side. Moreover, the term "front end" refers to an end of any member or part at the front end side while the term "base end" refers to an end of any member or part at the base end side.

### ADVANTAGEOUS EFFECTS OF INVENTION

The present invention can provide a dilator allowing the completion of hole expansion on a target object to be accurately detected.

### BRIEF DESCRIPTION OF DRAWINGS

Fig. 1 shows a schematic partial cutaway side view of the entire of a first embodiment of the present invention.
Fig. 2 shows an enlarged schematic partial cutaway side view of a key portion of a modified example of the first embodiment.
Fig. 3 shows a schematic front view of Fig. 2.
Fig. 4 shows an enlarged schematic side view of a key portion of a modified example of the first embodiment.
Fig. 5 shows an enlarged schematic side view of a key portion of a second embodiment of the present invention.
Fig. 6 shows an enlarged schematic side view of a key portion of a modified example of the second embodiment.
Fig. 7 shows a schematic partial cutaway side view of a key portion of a third embodiment of the present invention.
Fig. 8 shows an enlarged schematic side view of a key portion of a fourth embodiment of the present invention.

### DESCRIPTION OF ENBODIMENTS

Provided is a dilator including a hollow shaft having a tapered portion and a body portion having a front end located at a base end of the tapered portion, the tapered portion having an outer diameter of a front end smaller than that of the base end, the body portion extending toward a base end side in an axis direction of the tapered portion, in which a spirally-arranged protruding portion is provided on an outer peripheral surface of the tapered portion, and the spirally-arranged protruding portion has a gap along an axis direction of the shaft, and a base end of the spirally-arranged protruding portion is located at the base end of the tapered portion.

Below, the first to fourth embodiments of the present invention will be described with reference to the figures, but the present invention shall not only be limited to the embodiments shown in the accompanying figures. It is noted that the dimensions of dilators shown in the figures are provided to merely facilitate understanding of the embodiments, but do not necessarily correspond to the actual dimensions.

### First embodiment

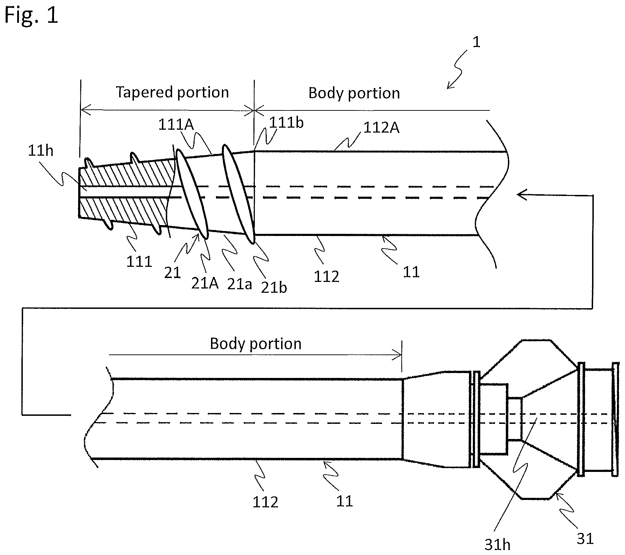

Fig. 1 shows a schematic partial cutaway side view of the entire of the first embodiment of the present invention. As shown in Fig. 1, a dilator 1 generally includes a shaft 11, a spirally-arranged protruding portion 21, and a base portion 31.

The shaft 11 is a member having an through-hole 11h of a hollow shape. A guide wire (not shown) and the like, for example, are to be inserted through the through-hole 11h. In order to allow them to be inserted freely, the through-hole 11h includes a continuous space connecting a front end and a base end of the shaft 11. The shaft 11 has a tapered portion 111 and a body portion 112.

The tapered portion 111 is a part having an outer diameter of a front end smaller than that of a base end. Specifically, the tapered portion 111 is connected to a front end of the body portion 112 described below, and extends from that front end toward the front end side, and has a shape tapered toward the front end side.

The body portion 112 is a part having a front end located at a base end 111b of the tapered portion 111 and extending toward the base end side in the axis direction of tapered portion 111. Specifically, the body portion 112 is, for example, connected to the base end 111b of the aforementioned tapered portion 111 at the front end, and also connected to the base portion 31 described below at the base end. The body portion 112 has a substantially constant outer diameter from the front end thereof through the base end. It is noted that the body portion 112 may be integrally formed with the tapered portion 111, or may be formed as a separate body. In the present embodiment, the body portion 112 is integrally formed with the tapered portion 111 by casting and the like.

Materials for the tapered portion 111 and the body portion 112 as described above preferably have antithrombogenicity, flexibility, and biocompatibility because they are to be inserted into a body cavity. Examples of the materials include resin materials such as polyamide resin, polyolefin resin, polyester resin, polyurethane resin, silicone resin, and fluororesin; metal materials such as stainless steel and superelastic alloys (nickel-titanium alloys); and the like. It is noted that the tapered portion 111 and the body portion 112 may have various types of coating on their surface sides. Examples of the coating include a protective film(s) (representative example: a plating film(s)) located at the surfaces of the tapered portion 111 and the body portion 112, an underlying film for improving adhesiveness of the tapered portion 111 and the body portion 112 with the spirally-arranged protruding portion 21, and the like.

The spirally-arranged protruding portion 21 is provided on an outer peripheral surface 111A of the tapered portion 111, and has a gap 21a along the axis direction of the shaft 11. Specifically, the above spirally-arranged protruding portion 21 can be formed, for example, as a single-thread or multi-thread protruded portion protruded radially and outwardly from the outer peripheral surface 111A of the tapered portion 111 (the outermost surface, outermost portion of the dilator 1), the single-thread or multi-thread protruded portion being continuous or discontinuous in the axis direction. Further, the spirally-arranged protruding portion 21 is arranged so that adjacent portions of the spirally-arranged protruding portion 21 are spaced apart in the axis direction. This enables the tapered portion 111 to be moved forward due to the screw action of the spirally-arranged protruding portion 21 by a rotational operation of the shaft 11, leading to smooth expansion of a hole with the tapered portion 111. It is noted that the spirally-arranged protruding portion 21 may be integrally formed with the shaft 11, or may be formed as a separate body. In the present embodiment, the spirally-arranged protruding portion 21 is integrally formed with the tapered portion 111 of the shaft 11 by casting and the like.

Here, a base end 21b of the spirally-arranged protruding portion 21 is located at the base end 111b of the tapered portion 111. That is, the spirally-arranged protruding portion 21 is provided on the outer peripheral surface 111A of the tapered portion 111 in the present embodiment.

It is noted that the spirally-arranged protruding portion 21 preferably does not serve as a blade (it does not have a shape where a living body tissue may be cut). That is, the spirally-arranged protruding portion 21 preferably does not have a sharp edge at an end portion in a radially outer side of the shaft on a cross-section (for example, on a cross-section perpendicular to the spiral direction of the spirally-arranged protruding portion). Such end portions can include, for example, a part having a shape including an obtuse edge or a curve (for example, a curve constituting a part of a circle or an ellipse), and the like. This enables the dilator 1 to expand a hole pre-formed on a target object (for example, the wall of the digestive tract such as the patient's stomach) without damaging living body tissues at the inner surface of the hole.

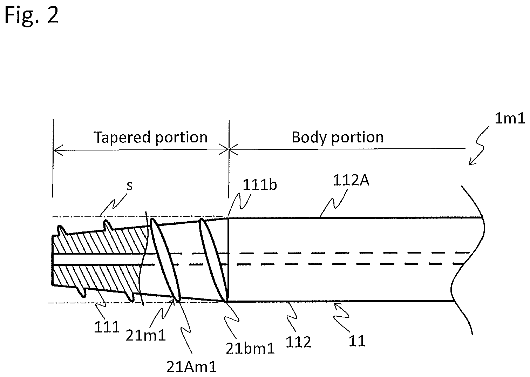

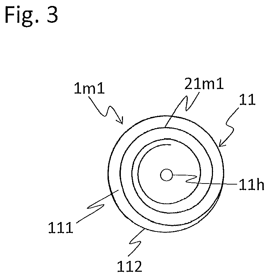

Further, according to the above dilator, the outer shape of the spirally-arranged protruding portion in a front view in the axis direction of the tapered portion 111 falls within the outer shape of the body portion 112 in a front view in the axis direction of the tapered portion 111 in a state where the tapered portion 111 and the body portion 112 extend in a substantially linear fashion. That is, as shown in Figs. 2 and 3, the shape (the outer shape) of an outer peripheral surface 21Am1 of a spirally-arranged protruding portion 21m1 viewed from the front end side along the axis of the shaft 11 preferably falls in a region within a virtual cylinder s (see a dilator 1m1). The virtual cylinder s is defined by virtual lines extending from the outer peripheral surface 112A (the outer shape) of the body portion 112 toward the front end side.

The outer shape of the spirally-arranged protruding portion 21m1 formed as described above can reduce the screwing-in resistance of the shaft 11 more noticeably (and thus can allow an operator to reliably perceive it) when expansion of a hole on a target object is completed. Therefore, the completion of hole expansion can be more accurately detected.

There is no particular limitation for a material of the spirally-arranged protruding portion 21 as long as the effects of the present invention are not impaired. The followings can be used: for example, metal materials such as stainless steel and superelastic alloys (nickel-titanium alloys), biocompatible resin materials such as polyamide resin and fluororesin, or the like. A radiopaque material can preferably be included. Such radiopaque materials include, for example, gold, platinum, tungsten, or alloys including these elements (for example, platinum-nickel alloys and the like), and the like. Further, a radiopaque material may be combined with a material other than the radiopaque material such as a radiopaque material to be coated on a non-radiopaque material.

When a material of the spirally-arranged protruding portion 21 contains a radiopaque material as described above, the position of the spirally-arranged protruding portion 21 can be perceived under radiographic imaging. This enables the completion of hole expansion to be detected more accurately.

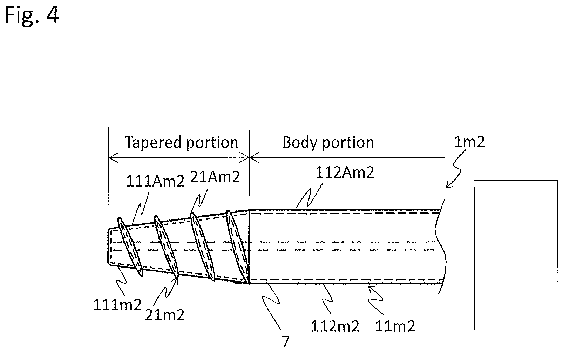

Here, the outer periphery or peripheries of the shaft and/or the spirally-arranged protruding portion described above may have a coating 7 as shown in Fig. 4. Such a coating may be formed for the purposes of, for example, improving slidability and anti-biting of a living body tissue. Materials which can be used to form the coating 7, if any, include, for example, biocompatible resin materials such as polyamide resin and fluororesin, hydrophilic coating materials, or the like. The above coating 7 may be configured to have a thickness of, for example, 0.1 µm to 300 µm. It is noted that when the shaft and/or the spirally-arranged protruding portion have/has the coating 7, the outer peripheral surface (the outer surface) of the coating 7 corresponds to an outer peripheral surface of a shaft 11m2 (an outer peripheral surface 111Am2 of the tapered portion 111m2 and an outer peripheral surface 112Am2 of a body portion 112m2) or an outer peripheral surface 21Am2 of a spirally-arranged protruding portion 21m2 (see a dilator 1m2).

The base member 31 as shown in Fig. 1 is a part with which an operator pushes the dilator 1 into the body, and/or performs a rotation operation. The above base member 31 has a front end connected to the base end of the body portion 112 and an through-hole 31h in communication with the through-hole 11h of the shaft 11. During the procedure, a guide wire and the like will be inserted through the through-hole 31h of the base member 31.

The length of each portion of the dilator 1 in the axis direction is usually as follows: 1,600 mm to 2,500 mm for the entire shaft 11 and 5 mm to 100 mm for the tapered portion 111. The outer diameter of each portion of the shaft 11 is usually as follows: 0.6 mm to 2.3 mm for the front end of the tapered portion 111 and 1.2 mm to 4.0 mm for the base end 111b of the tapered portion 111 and the body portion 112. The inner diameter of the through-hole 11h of the shaft 11 is usually 0.4 mm to 1.0 mm.

The length of each portion of the dilator 1 according to the present embodiment in the axis direction is as follows: 2,000 mm for the entire shaft 11 and 30 mm for the tapered portion 111. The outer diameter of each portion of the shaft 11 is as follows: 1.1 mm for the front end of the tapered portion 111 and 1.9 mm for the base end 111b of the tapered portion 111 and the body portion 112. The inner diameter of the through-hole 11h of the shaft 11 is 0.7 mm.

Next, an example of operating modes of the above dilator 1 will be described.

First, a target object is punctured with an introducer needle to open a hole. Subsequently, a guide wire (not shown) is inserted into an through-hole of the introducer needle, and then the introducer needle is withdrawn.

Next, the base end of the guide wire is inserted into the through-hole 11h, 31h of the dilator 1, and the dilator 1 is then inserted. Subsequently, the dilator 1 is pushed in while rotating the shaft 11 to expand the hole at a punctured portion. During this, the tapered portion 111 can be moved forward to the front end side due to the screw action and the like of the spirally-arranged protruding portion 21 by a rotation operation of the shaft 11, leading to smooth hole expansion with the tapered portion 111. It is noted that the screwing-in resistance of the shaft 11 suddenly decreases when the base end 111b of the tapered portion 111 passes through a punctured portion and hole expansion on a target object is completed (when the hole expansion at the punctured portion is completed) because the spirally-arranged protruding portion 111 is not provided on the outer peripheral surface 112A of the body portion 112. This can allow an operator to accurately detect when the hole expansion is completed.

As described above, the dilator 1 having the aforementioned configuration can reduce the screwing-in resistance of the shaft 11 when expansion of a hole on a target object is completed, allowing an operator to accurately detect when the hole expansion is completed by means of a sensuous communication received by the operator.

### Second embodiment

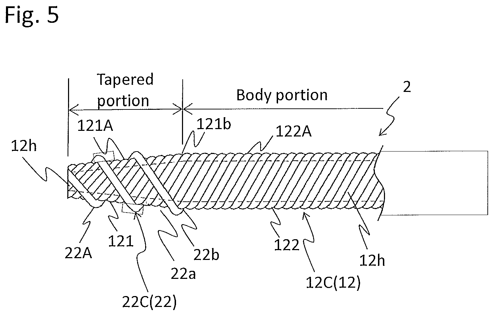

Fig. 5 shows an enlarged schematic side view of a key portion of the second embodiment of the present invention. As shown in Fig. 5, a dilator 2 generally includes a shaft 12, a spirally-arranged protruding portion 22, and the base portion 31 (see Fig. 1). The dilator 2 differs from the first embodiment in the configurations of the shaft 12 and the spirally-arranged protruding portion 22. It is noted that the configurations other than those of the shaft 12 and the spirally-arranged protruding portion 22 and the operating modes of the dilator 2 are similar to those of the first embodiment. Therefore, the same reference symbol is given to the same part, and detailed description thereof will be omitted.

The shaft 12 is a member having an through-hole 12h of a hollow shape. The shaft 12 has a tapered portion 121 and a body portion 122. The tapered portion 121 is a part where the outer diameter is smaller at the front end than at the base end. The body portion 122 is a part having a front end located at a base end 121b of the tapered portion 121 and extending toward the base end side in the axis direction of tapered portion 121.

The shaft 12 according to the present embodiment is formed by winding a wire around the axis of the above shaft 12. Specifically, the above shaft 12 is formed as a coil body 12C in which a single wire is spirally wound around so that adjacent portions of the wire are brought into close contact with each other in the axis direction as shown in Fig. 5. The coil body 12C serves as the aforementioned tapered portion 121 and the aforementioned body portion 122, depending on the shape of the outer peripheral surface thereof. It is noted that in Fig. 5, the common inscribed lines of the coil body 12C are shown by dotted lines, and the through-hole 12h is formed in a region surrounded by the common inscribed lines.

The spirally-arranged protruding portion 22 is provided on an outer peripheral surface 121A of the tapered portion 121, and has a gap 22a along the axis direction of the shaft 12. Further, a base end 22b of the spirally-arranged protruding portion 22 is located at the base end 121b of the tapered portion 121.

The spirally-arranged protruding portion 22 according to the present embodiment is formed by winding a wire around on the outer peripheral surface of the shaft 12 (the outer peripheral surface 121A of the tapered portion 121). Specifically, the above spirally-arranged protruding portion 22 is, for example, formed as a coil body 22C in which a single wire is spirally wound around so that adjacent portions of the wire are spaced apart from each other. The inner periphery of the coil body 22C is brought into close contact with the outer periphery of the coil body 12C, and both the coil body 22C and the coil body 12C are joined to each other, for example, by soldering or welding each end of the both, fixing each end of the both via an adhesive, or fusing each end of the both via coating.

The number of wires used for the shaft 12 and the spirally-arranged protruding portion 22 may be one or more. It is noted that the winding directions of wires of the shaft 12 and the spirally-arranged protruding portion 22 are preferably opposite to each other as in the present embodiment. That is, preferably, one of the coil body 12C and the coil body 22C is S-twisted, and the other is Z-twisted. By this configuration, the direction of a force axially applied to a wire of the shaft 12 can be directed as opposed to the direction of a force axially applied to a wire of the spirally-arranged protruding portion 22, preventing decreases of torquability and pushability due to separation of adjacent portions of a wire of the shaft 12.

As materials for a wire of the aforementioned coil body 12C, for example, materials similar to those listed as materials of the tapered portion and the body portion in the first embodiment can be exemplified. As materials for a wire of the aforementioned coil body 22C, for example, materials similar to those listed as materials of the spirally-arranged protruding portion 22 in the first embodiment can be exemplified.

As described above, the dilator 2 includes the shaft 12 and the spirally-arranged protruding portion 22 which are formed with the coil body 12C, 22C, respectively. This configuration can improve the flexibility and torquability of the shaft 12 and the spirally-arranged protruding portion 22.

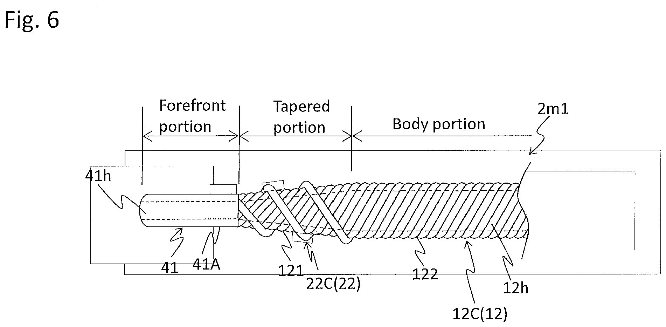

It is noted that a forefront portion 41 having a substantially tubular and hollow shape may be attached to the front end of the shaft 12 as shown in Fig. 6. The forefront portion 41 has a surface 41A flatter than the outer peripheral surface of the coil 12C (an uneven surface due to the presence of a wire) and an through-hole 41h in communication with the through-hole 12h of the shaft 12. The above forefront portion 41 may be formed so as to have a surface smoothed by applying a soldering material such as silver-tin solder and gold-tin solder and the like to the front end portion of the coil body 12C. This can improve the insertability of the shaft into a hole opened with an introducer needle, enabling the procedure to be performed smoothly (see a dilator 2m1).

### Third embodiment

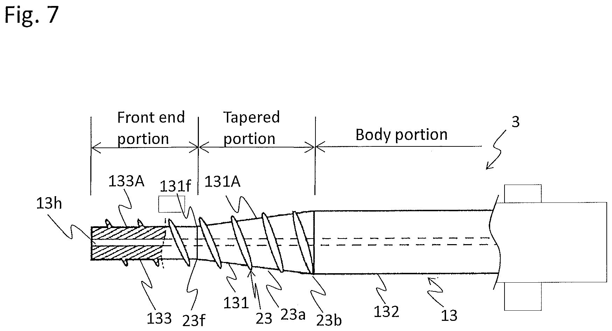

Fig. 7 shows a schematic partial cutaway side view of a key portion of the third embodiment of the present invention. As shown in Fig. 7, a dilator 3 generally includes a shaft 13, a spirally-arranged protruding portion 23, and the base portion 31 (see Fig. 1). The dilator 3 differs from the first embodiment in the configurations of the shaft 13 and the spirally-arranged protruding portion 23. It is noted that the configurations other than those of the shaft 13 and the spirally-arranged protruding portion 23 and the operating modes of the dilator 3 are similar to those of the first embodiment. Therefore, the same reference symbol is given to the same part, and detailed description thereof will be omitted.

The shaft 13 is a member having an through-hole 13h of a hollow shape. The shaft 13 has a tapered portion 131, a body portion 132, and a front end portion 133. It is noted that the tapered portion 131 and the body portion 132 have the same configurations as in the first embodiment, and the descriptions thereof will be applied.

The front end portion 133 is a part having a base end located at a front end 131f of the tapered portion 131 of the shaft 13 and extending toward the front end side in the axis direction of the tapered portion 131. Specifically, the front end portion 133 has, for example, a substantially constant outer diameter from the front end thereof through the base end as shown in Fig. 7. It is noted that the front end portion 133 may be integrally formed with the tapered portion 131, or may be formed as a separate body. In the present embodiment, the front end portion 133 is integrally formed with the tapered portion 131 by casting and the like.

As materials for the front end portion 133, for example, materials similar to those listed as materials for the tapered portion and the body portion in the first embodiment can be exemplified.

The spirally-arranged protruding portion 23 is provided on outer peripheral surfaces 133A and 131A of the front end portion 133 and the tapered portion 131, and has a gap 23a along the axis direction of the shaft 13. It is noted that the spirally-arranged protruding portion 23 is formed as a continuous single thread in the present embodiment. Moreover, the spirally-arranged protruding portion 23 is integrally formed with the front end portion 133 and the tapered portion 131 by casting, and the like.

Here, the front end of the spirally-arranged protruding portion 23 is preferably located at the front end of the tapered portion 131 or the outer peripheral surface 133A of the front end portion 133. In the present embodiment, the front end of the spirally-arranged protruding portion 23 is located at the front end of the front end portion 133, and the base end is located at the base end of the tapered portion 131. Thereby, a large driving force can be obtained from the spirally-arranged protruding portion 23 when the shaft 13 is rotated, leading to more reliable hole expansion.

As described above, the front end portion 133 included in the dilator 3 can be pre-inserted into a hole before expanding the hole with the tapered portion 131, leading to stable and reliable hole expansion with the tapered portion 131.

### Fourth embodiment

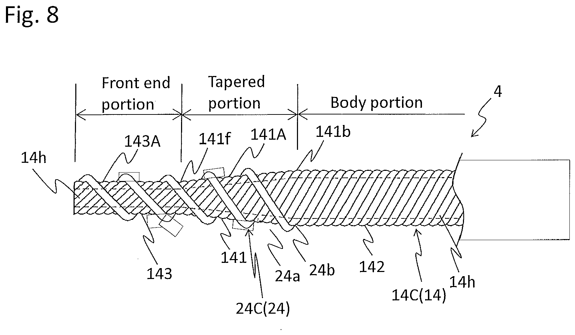

Fig. 8 shows an enlarged schematic side view of a key portion of the fourth embodiment of the present invention. As shown in Fig. 8, a dilator 4 generally includes a shaft 14, a spirally-arranged protruding portion 24, and the base portion 31 (see Fig. 1). The dilator 4 differs from the third embodiment in the configurations of the shaft 14 and the spirally-arranged protruding portion 24. It is noted that the configurations other than those of the shaft 14 and the spirally-arranged protruding portion 24 and the operating modes of the dilator 4 are similar to those of the first embodiment. Therefore, the same reference symbol is given to the same part, and detailed description thereof will be omitted.

The shaft 14 is a member having an through-hole 14h of a hollow shape. The shaft 14 has a tapered portion 141, a body portion 142, and a front end portion 143. The tapered portion 141 is a part where the outer diameter is smaller at the front end than at the base end. The body portion 142 is a part having a front end located at a base end 141b of the tapered portion 141 and extending toward the base end side in the axis direction of the tapered portion 141. The front end portion 143 is a part having a base end located at a front end 141f of the tapered portion 141 in the shaft 14 and extending toward the front end side in the axis direction of the tapered portion 141.

The shaft 14 according to the present embodiment is formed by winding a wire around the axis of the shaft 14. Specifically, the shaft 14 is formed with a coil body 14C, and can serve as the tapered portion 141, the body portion 142, and the front end portion 143, depending on the shape of the outer peripheral surface thereof. It is noted that in Fig. 8, the common inscribed lines of the coil body 14C are shown by dotted lines, and the through-hole 14h is formed in a region surrounded by the common inscribed lines.

The spirally-arranged protruding portion 24 is provided on outer peripheral surfaces 143A and 141A of the front end portion 143 and the tapered portion 141, and has a gap 24a along the axis direction of the shaft 14. Further, a base end 24b of the spirally-arranged protruding portion 24 is located at the base end 141b of the tapered portion 141.

The spirally-arranged protruding portion 24 according to the present embodiment is formed by winding a wire around on the outer peripheral surface of the shaft 14 (the outer peripheral surface 143A of the front end portion 143 and the outer peripheral surface 141A of the tapered portion 141). Specifically, the above spirally-arranged protruding portion 24 is, for example, formed as a coil body 24C in which a single wire is spirally wound around so that adjacent portions of the wire are spaced apart from each other.

It is noted that a method of joining the coil body 24C with the coil body 14C, a material for a wire of each coil body, and the like are similar to those of the second embodiment, and the descriptions thereof will be applied.

As described above, the dilator 4 includes the shaft 14 and the spirally-arranged protruding portion 24 which are formed with the coil body 14C, 24C, respectively. This configuration can improve the flexibility and torquability of the shaft 14 and the spirally-arranged protruding portion 24. Further, the front end portion 143 included in the dilator 4 can be pre-inserted into a hole before expanding the hole with the tapered portion 141, leading to stable and reliable hole expansion with the tapered portion 141.

It is noted that the present invention shall not be limited to the configurations of the aforementioned embodiments. All of alternations made within the scope of the claims and within the meaning and range equivalent to the scope of the claims are intended to be included.

For example, the dilators 1 to 4 are described in which the front ends of the spirally-arranged protruding portions 21 and 22 are located at the front ends of the tapered portions 111 and 121 in the aforementioned first and second embodiments, and the front ends of the spirally-arranged protruding portions 23 and 24 are located at the front ends of the front end portions 133 and 143 in the third and fourth embodiments. However, a dilator having a front end portion may be formed such that the front end of a spirally-arranged protruding portion is located at a part between the front end of a front end portion and the base end of a tapered portion, and a dilator without having a front end portion may be formed such that the front end of a spirally-arranged protruding portion is located at a part between the front end and the base end of a tapered portion.

Moreover, the dilator 2 is described in the aforementioned second embodiment where the shaft 12 and the spirally-arranged protruding portion 22 are formed with the coil body 12C, 22C, respectively, and the dilator 4 is described in the aforementioned fourth embodiment where the shaft 14 and the spirally-arranged protruding portion 24 are formed with the coil body 14C, 24C, respectively. However, a dilator formed with a coil body may be configured such that only either one of a shaft and a spirally-arranged protruding portion is formed with a coil body.

Moreover, the dilator 2 is described in the aforementioned second embodiment where the coil body 12C and the coil body 22C are each formed with a continuous single thread of wire, and the dilator 4 is described in the aforementioned fourth embodiment where the coil body 14C and the coil body 24C are each formed with a continuous single thread of wire. However, a dilator may include coil bodies each including a multi-thread coil formed with a plurality of wires, or a dilator may include intermittently-wound-around coil bodies.

Further, the dilators 1 to 4 having no coating on the surfaces of the shafts 11 to 14 are shown in the aforementioned embodiments (see Figs. 1 to 8). However, a dilator may have various types of coating on the surface side of a shaft (including a part between the shaft and a spirally-arranged protruding portion). Examples of the coating include, for example, a protective film for protecting the surface of the shaft (representative example: a plating film), an underlying film for improving adhesiveness between the shaft and the spirally-arranged protruding portion, and the like.

### REFERENCE SIGNS LIST

- 1 to 4: Dilator
- 11 to 14: Shaft
- 21 to 24: Spirally-arranged protruding portion
- 111, 121, 131, 141: Tapered portion
- 112, 122, 132, 142: Body portion
- 133, 143: Front end portion

## Claims

1. A dilator comprising:
a hollow shaft having a tapered portion and a body portion having a front end located at a base end of the tapered portion, the tapered portion having an outer diameter of a front end smaller than that of the base end, the body portion extending toward a base end side in an axis direction of the tapered portion, wherein,
a spirally-arranged protruding portion is provided on an outer peripheral surface of the tapered portion, and
the spirally-arranged protruding portion has a gap along an axis direction of the shaft, and
a base end of the spirally-arranged protruding portion is located at the base end of the tapered portion.

2. The dilator according to claim 1, wherein
the outer shape of the spirally-arranged protruding portion in a front view in the axis direction of the tapered portion falls within the outer shape of the body portion in a front view in the axis direction of the tapered portion in a state where the tapered portion and the body portion extend in a substantially linear fashion.

3. The dilator according to claim 1 or 2, wherein
a material of the spirally-arranged protruding portion includes a radiopaque material.

4. The dilator according to any one of claims 1 to 3, wherein
the shaft includes a front end portion having a base end located at the front end of the tapered portion and extending toward a front end side in the axis direction of the tapered portion.

5. The dilator according to claim 4, wherein
a front end of the spirally-arranged protruding portion is located at the front end of the tapered portion or an outer peripheral surface of the front end portion.

6. The dilator according to any one of claims 1 to 5, wherein
the shaft is formed such that a wire is wound around an axis of the shaft.

7. The dilator according to any one of claims 1 to 6, wherein
the spirally-arranged protruding portion is formed such that a wire is wound around on an outer peripheral surface of the shaft.

8. The dilator according to any one of claims 1 to 5, wherein
the shaft is formed such that a wire is wound around the axis of the shaft, and the spirally-arranged protruding portion is formed such that a wire is wound around on the outer peripheral surface of the shaft, and
a winding direction of the wire of the shaft and a winding direction of the wire of the spirally-arranged protruding portion are opposite to each other.
